(19) Europäisches Patentamt
European Patent Office
Office européen des brevets

(11) Veröffentlichungsnummer: **0 192 150**
**A2**

(12) # EUROPÄISCHE PATENTANMELDUNG

(21) Anmeldenummer: **86101653.3**

(22) Anmeldetag: **10.02.86**

(51) Int. Cl.[4]: **C 07 C 127/24**
C 07 C 127/22, C 07 C 155/02
C 07 D 295/20, A 01 N 47/34
A 01 N 47/38

(30) Priorität: **22.02.85 DE 3506236**

(43) Veröffentlichungstag der Anmeldung:
**27.08.86 Patentblatt 86/35**

(84) Benannte Vertragsstaaten:
**AT BE CH DE FR GB IT LI NL**

(71) Anmelder: **BAYER AG**
**Konzernverwaltung RP Patentabteilung**
**D-5090 Leverkusen 1 Bayerwerk(DE)**

(72) Erfinder: **Führer, Wolfgang, Dr.**
**Wehrstrasse 28**
**D-5202 Hennef(DE)**

(72) Erfinder: **Kühle, Engelbert, Dr.**
**von Bodelschwingh-Strasse 42**
**D-5060 Bergisch Gladbach 2(DE)**

(72) Erfinder: **Schlee, Hans-Georg, Dr.**
**Florastrasse 25**
**D-5090 Leverkusen 3(DE)**

(72) Erfinder: **Zumach, Gerhard, Dr.**
**Morgengraben 6**
**D-5000 Koeln 80(DE)**

(72) Erfinder: **Eue, Ludwig, Dr.**
**Paul-Klee-Strasse 36**
**D-5090 Leverkusen 1(DE)**

(72) Erfinder: **Schmidt, Robert R., Dr.**
**Im Waldwinkel 110**
**D-5060 Bergisch Gladbach(DE)**

(72) Erfinder: **Homeyer, Bernhard, Dr.**
**Obere Strasse 28**
**D-5090 Leverkusen 3(DE)**

(72) Erfinder: **Reinecke, Paul, Dr.**
**Steinstrasse 8**
**D-5090 Leverkusen 3(DE)**

(72) Erfinder: **Hänssler, Gerd, Dr.**
**Am Arenzberg 58a**
**D-5090 Leverkusen 3(DE)**

(72) Erfinder: **Santel, Hans-Joachim, Dr.**
**Grünstrasse 9a**
**D-5090 Leverkusen 1(DE)**

(54) **1-Acyl-1-phenyl-harnstoffe.**

(57) Es werden neue 1-Acyl-1-phenylharnstoffe der Formel

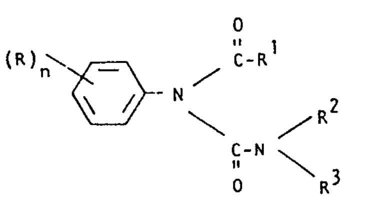

(I)

bereitgestellt, in welcher die Reste

R gleich oder verschieden sind und für Halogen, Alkyl, Alkoxy, Halogenalkyl, Halogenalkoxy, Alkylthio, Halogenalkylthio oder Nitro stehen,

n für 0, 1, 2, 3, 4 oder 5 steht,

$R^1$ für Alkoxy, Alkenoxy, Alkinoxy, Alkoxyalkoxy, Alkylthio, Arylthio, Alkylamino, Dialkylamino, Cycloalkylamino oder für einen über Stickstoff gebundenen N-Heterocyclus steht,

$R^2$ für Wasserstoff, Alkyl, Alkenyl oder Alkoxy steht und

$R^3$ für Alkyl, Alkenyl, Cycloalkyl oder Aralkyl steht oder

$R^2$ und $R^3$ gemeinsam mit dem Stickstoffatom, an das sie gebunden sind, für einen heterocyclischen Rest stehen, und in welcher der Rest $R^1$ mit dem Rest

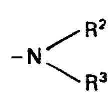

identisch sein kann.

Die neuen Verbindungen zeigen hervorragende biologische, insbesondere herbizide, fungizide und insektizide Eigenschaften.

EP 0 192 150 A2

0192150

BAYER AKTIENGESELLSCHAFT 5090 Leverkusen, Bayerwerk

Konzernverwaltung RP Bi/by-c
Patentabteilung Ib

21. FEB. 1985

# 1-Acyl-1-phenyl-harnstoffe

Die Erfindung betrifft neue 1-Acyl-1-phenyl-harnstoffe, Verfahren zu ihrer Herstellung und ihre Verwendung als Pflanzenschutz- bzw. Pflanzenbehandlungsmittel, insbesondere als selektive Herbizide, Fungizide und Insektizide.

Es ist bekannt, daß sich verschiedene substituierte N-Arylharnstoffe, z.B. N-(3,4-Dichlorphenyl)-N',N'-dimethylharnstoff (Diuron), zur Bekämpfung unerwünschten Pflanzenwuchses eignen (vgl. z.B. R. Wegler, Chemie der Pflanzenschutz- und Schädlingsbekämpfungsmittel, Band 2, Seite 241 ff., Springer Verlag Berlin 1970). Die Verträglichkeit derartiger Verbindungen ist aber bei Aufwandmengen, die für eine ausreichende Unkrautbekämpfung erforderlich sind, in verschiedenen Kulturen nicht in jedem Fall zufriedenstellend.

Es wurden nun neue 1-Acyl-1-phenyl-harnstoffe der allgemeinen Formel (I) gefunden,

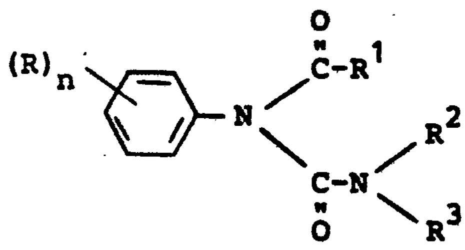

(I)

in welcher die Reste

R gleich oder verschieden sind und für Halogen, Alkyl, Alkoxy, Halogenalkyl, Halogenalkoxy, Alkylthio, Halogenalkylthio oder Nitro stehen,

n für 0, 1, 2, 3, 4 oder 5 steht,

$R^1$ für Alkoxy, Alkenoxy, Alkinoxy, Alkoxyalkoxy, Alkylthio, Arylthio, Alkylamino, Dialkylamino, Cycloalkylamino oder für einen über Stickstoff gebundenen N-Heterocyclus steht,

$R^2$ für Wasserstoff, Alkyl, Alkenyl oder Alkoxy steht und

$R^3$ für Alkyl, Alkenyl, Cycloalkyl oder Aralkyl steht oder

$R^2$ und $R^3$ gemeinsam mit dem Stickstoffatom, an das sie gebunden sind, für einen heterocyclischen Rest stehen, und in welcher der Rest $R^1$ mit dem Rest

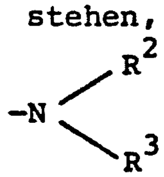

identisch sein kann.

0192150

Weiterhin wurde gefunden, daß man die neuen 1-Acyl-1-phenyl-harnstoffe der allgemeinen Formel (I) erhält, wenn man

(a) N-Acyl-N-chlorcarbonyl-aniline der Formel (II)

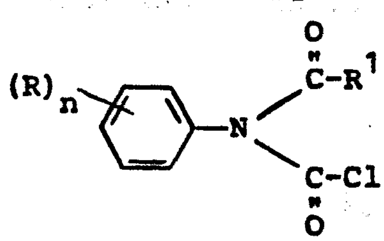

(II)

in welcher

R, n und $R^1$ die oben angegebene Bedeutung besitzen,

mit Aminen der Formel (III)

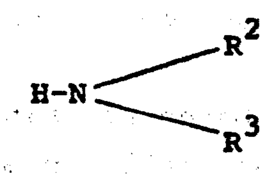

(III)

in welcher

$R^2$ und $R^3$ die oben angegebene Bedeutung haben,

gegebenenfalls in Gegenwart eines Verdünnungsmittels und gegebenenfalls in Gegenwart eines Säurebindemittels, umsetzt,

oder

(b) zur Herstellung von Verbindungen der Formel (I), in welcher die Reste $R^1$ und

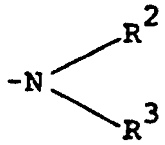

voneinander verschieden sind, ein Mol einer N-Phenylimido-dicarbonylverbindung der Formel (IV)

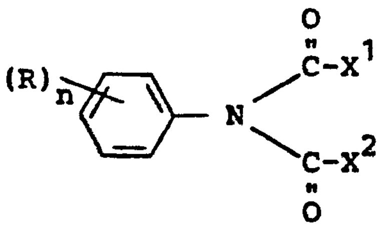

(IV)

in welcher

R und n die oben angegebene Bedeutung haben und

$X^1$ und $X^2$ gleich oder verschieden sein können und für Halogen oder Phenoxy stehen,

gegebenenfalls in Gegenwart eines Verdünnungsmittels und gegebenenfalls in Gegenwart einer Hilfsbase in beliebiger Reihenfolge mit etwa einem Mol einer Verbindung der Formel

$$M-R^1 \quad (V)$$

in welcher

$R^1$ die oben angegebene Bedeutung hat und

M für Wasserstoff oder ein Äquivalent eines Metallkations steht,

und etwa einem Mol einer Verbindung der Formel (III)

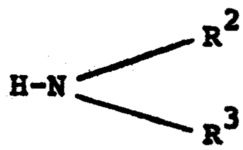 (III)

worin

$R^2$ und $R^3$ die oben angegebene Bedeutung haben, umsetzt,

oder

(c) zum Erhalt von Verbindungen der Formel (I), in welcher der Rest $R^1$ mit dem Rest

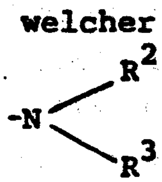 identisch ist, ein Mol einer Verbindung

der Formel

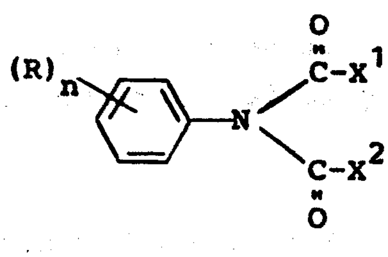 (IV)

in welcher

R, n, $X^1$ und $X^2$ die oben angegebene Bedeutung besitzen,

mit mindestens zwei Mol einer Verbindung der Formel

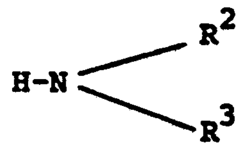

(III)

worin

$R^2$ und $R^3$ die oben angegebene Bedeutung haben,

gegebenenfalls in Gegenwart eines Verdünnungsmittels und gegebenenfalls in Gegenwart eines Säurebindemittels umsetzt.

Die neuen 1-Acyl-1-phenyl-harnstoffe der Formel (I) weisen hervorragende herbizide Eigenschaften auf und zeigen gegenüber den zugrundeliegenden Arylharnstoffen, wie z.B. dem N-(3,4-Dichlorphenyl)-N,N'-dimethylharnstoff (Diuron), wesentlich verbesserte Anwendungsmöglichkeiten auf.

Außerdem zeigen die neuen 1-Acyl-1-phenyl-harnstoffe der Formel (I) gute fungizide und insektizide Eigenschaften.

Die erfindungsgemäßen neuen Wirkstoffe können daher als Pflanzenschutz- bzw. Pflanzenbehandlungsmittel eingesetzt werden.

Die erfindungsgemäßen neuen N-Acyl-N-arylharnstoffe sind durch die Formel (I) allgemein definiert.

In dieser Formel steht vorzugsweise

R für Fluor, Chlor, Brom, geradkettiges oder verzweigtes Alkyl mit bis zu 12 C-Atomen, geradkettiges oder verzweigtes Alkoxy mit 1 bis 4 Kohlenstoffatomen, Halogenalkyl mit 1 bis 4 C-Atomen und einem oder mehreren F-, Cl- oder Br-Atomen, Halogenalkoxy mit 1 bis 4 C-Atomen und bis zu 9 gleichen oder verschiedenen Halogenatomen F, Cl, Br; Halogenalkylthio mit 1 bis 4 C-Atomen und gleichen oder verschiedenen Halogensubstituenten F, Cl, Br; oder für Nitro.

In der Formel (I) steht n für 0 bis 5, wobei die Substituenten R gleich oder verschieden sein können.

In der Formel (I) steht

$R^1$ vorzugsweise für Alkoxy ($C_1$-$C_{12}$), Alkenoxy ($C_2$-$C_4$), Alkinoxy ($C_2$-$C_4$), Alkoxyalkoxy mit jeweils 1 bis 4 C-Atomen im Alkoxyteil, Alkylthio ($C_1$-$C_6$), gegebenenfalls durch Chlor oder Methyl substituiertes Phenylthio, geradkettiges oder verzweigtes Alkylamino mit 1 bis 12 C-Atomen, geradkettiges oder verzweigtes Dialkylamino mit 1 bis 12 C-Atomen, wobei bei den Alkylamino- und Dialkylaminoderivaten auch ein oder beide Alkylreste einen carbocyclischen Ring mit 5 bis 7 C-Atomen bilden können, für einen über

Stickstoff gebundenen N-Heterocyclus mit 4 bis 8 Ringgliedern, die gegebenenfalls durch ein Sauerstoffatom unterbrochen sind,

$R^2$ steht in der Formel (I) vorzugsweise für Wasserstoff, Alkyl ($C_1$-$C_{12}$), Alkenyl ($C_1$-$C_4$), Alkoxy ($C_1$-$C_4$),

$R^3$ steht in der Formel (I) vorzugsweise für Alkyl ($C_1$-$C_{12}$), Alkenyl ($C_1$-$C_4$), Cycloalkyl mit 4 bis 8 Ring-C-Atomen, Arylalkyl mit 1 bis 5 C-Atomen im Alkylteil und 6-C-Atomen im Arylteil;

weiterhin stehen in der Formel (I) $R^2$ und $R^3$ gemeinsam mit dem Stickstoffatom, an das sie gebunden sind, vorzugsweise für einen N-Heterocyclus mit 4 bis 8 Ringgliedern, die gegebenenfalls auch Sauerstoff enthalten können.

Besonders bevorzugt sind in Formel (I) folgende Restebedeutungen R, $R^1$, $R^2$, $R^3$,

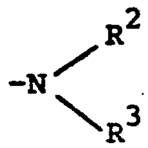 und n:

R = Wasserstoff, Fluor, Chlor, Brom, Methyl, Ethyl, Propyl, Isopropyl, Methoxy, Ethoxy, Propoxy, Isopropoxy, $CH_2Cl$, $CCl_3$, $CFCl_2$, $CF_2Cl$, $CF_3$, $OCF_3$, $SCF_3$, $NO_2$;

$R^1$ = geradkettiges oder verzweigtes Alkoxy ($C_1$-$C_{10}$), Allyloxy, Propargyloxy, Alkoxy($C_1$-$C_3$)alkoxy($C_1$-$C_2$), Alkylthio ($C_1$-$C_4$), Phenylthio, 4-Methylphenylthio, 4-Chlorphenylthio, Alkylamino ($C_1$-$C_{12}$), Cyclopentyl, Cyclohexyl, Pyrrolidino, Piperidino, Hexamethylenimino oder Morpholino;

$R^2$ = Wasserstoff, Methyl, Ethyl, Propyl, Isopropyl, Butyl, Isobutyl, tert.-Butyl, Pentyl, Isopentyl, Neopentyl, Allyl, Methoxy, Ethoxy;

$R^3$ = Methyl, Ethyl, Propyl, Isopropyl, Butyl, Isobutyl, tert.-Butyl, Pentyl, Isopentyl, Neopentyl, Allyl, Methoxy, Ethoxy, Cyclopentyl, Cyclohexyl;

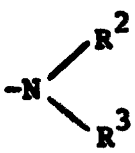 = Pyrrolidino, Piperidino, Hexamethylenimino oder Morpholino;

n = 0, 1, 2, 3, 4.

Verwendet man N-(4-Trifluormethylphenyl)-N-chlorcarbonylmethylurethan und Hexamethylenimin als Ausgangsstoffe, so kann der Verlauf des erfindungsgemäßen Verfahrens (a) durch das folgende Formelschema beschrieben werden:

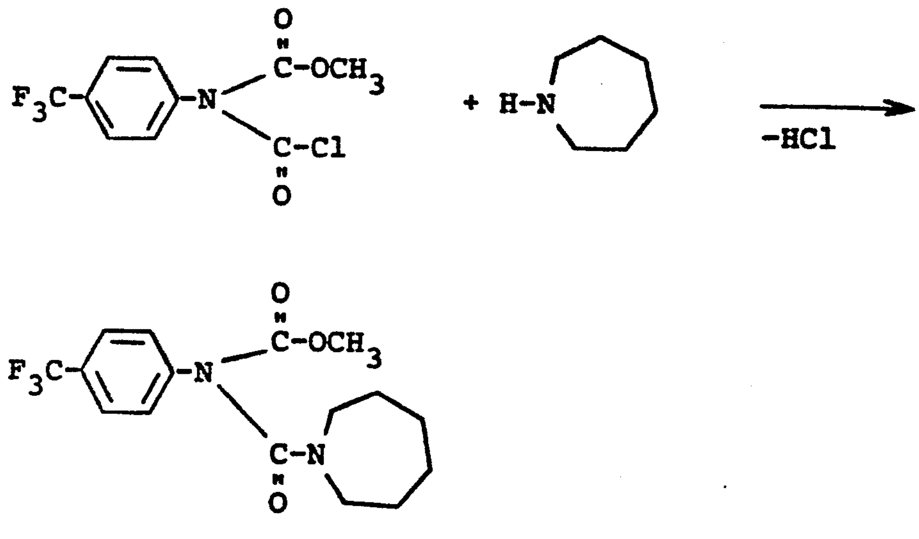


Verwendet man im Sinne der Herstellungsvariante (b) N-(4-Trifluormethylthiophenyl)-N,N-bischlorcarbonyl-anilin, $NaOCH_3$ und Dimethylamin als Ausgangskomponenten, so kann der Reaktionsverlauf durch das folgende Formelschema beschrieben werden:

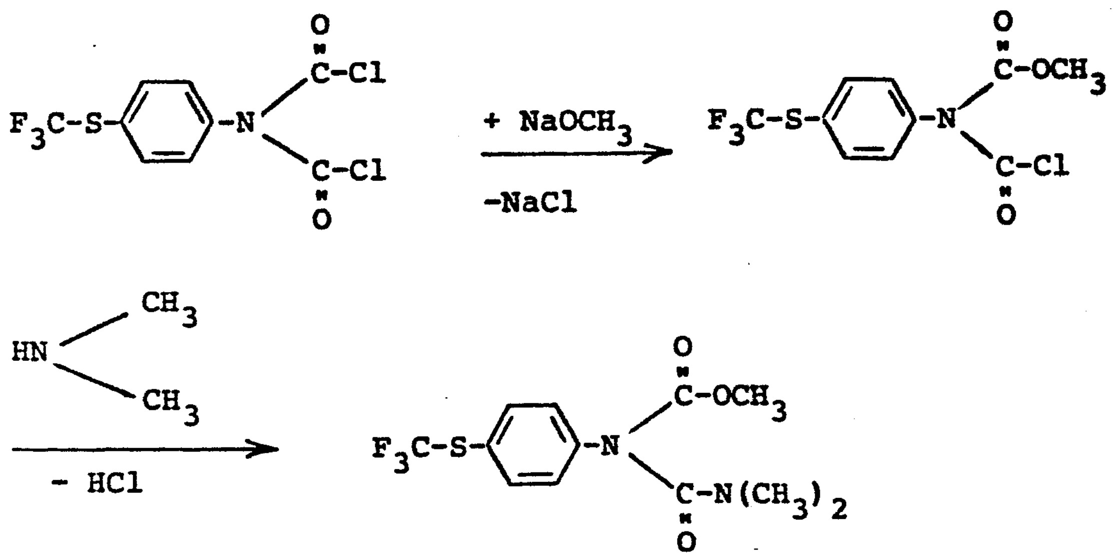

Verwendet man im Sinne der Herstellungsvariante (c) 4-Methoxy-bis-phenoxycarbonylanilin und Diethylamin als Ausgangsstoffe, so kann der Reaktionsverlauf durch das folgende Formelschema beschrieben werden:

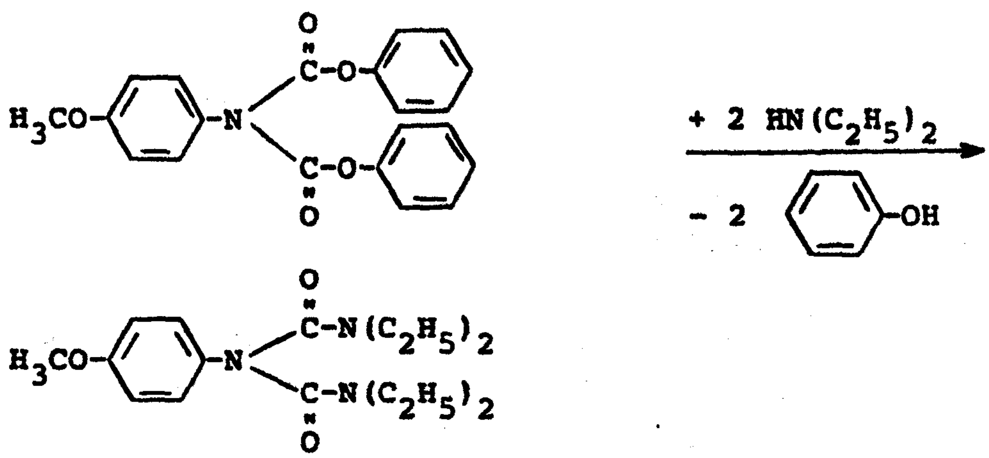


Die beim erfindungsgemäßen Verfahren (a) als Ausgangsprodukte benötigten N-Chlor-carbonylverbindungen der Formel (II) sind vorstehend definiert. Hierbei besitzen die Reste R, $R^1$ sowie n die unter Formel (I) angegebene Bedeutung.

Die N-Chlorcarbonylverbindungen der Formel (II) sind entweder aus der DE-OS 22 57 344 bekannt oder lassen sich nach dem dort beschriebenen Verfahren herstellen.

Man erhält die N-Chlorcarbonylverbindungen der Formel (II) zum Beispiel dadurch, daß man Kohlensäurederivate der Formel (VI)

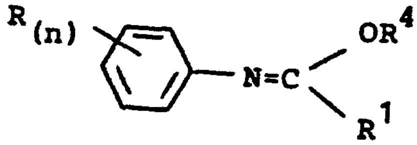

(VI)

in welcher

R, $R^1$ und n die obengenannte Bedeutung besitzen und

$R^4$ einen niederen Alkylrest, vorzugsweise Methyl oder Ethyl bedeutet,

mit Phosgen umsetzt, vorzugsweise in Lösungsmitteln wie Cyclohexan, Toluol oder Chlorbenzol, bei Temperaturen zwischen 20 und 120°C.

Die Kohlensäurederivate der Formel (VI) sind bekannt (vgl. z.B. E. Kühle et al., Angew. Chem. 81 (1969), S. 18 ff.).

Die Ausgangsstoffe der Formel (III) sind ebenfalls oben definiert. Hierbei besitzen die Reste $R^2$ und $R^3$ die unter Formel (I) angegebene Bedeutung. Die Verbindungen der Formel (III) sind allgemein bekannte Verbindungen der organischen Chemie.

Das erfindungsgemäße Verfahren (a) wird gegebenenfalls unter Verwendung von Verdünnungsmitteln durchgeführt. Als Verdünnungsmittel kommen hierbei praktisch alle inerten, aprotischen organischen Solventien in Frage.

Vorzugsweise verwendbar sind aliphatische und aromatische, gegebenenfalls halogenierte Kohlenwasserstoffe, wie Pentan, Hexan, Heptan, Cyclohexan, Benzol, Toluol, Xylol, Methylenchlorid, Chloroform, Tetrachlorkohlenstoff, Chlorbenzol und Dichlorbenzol, Ether, wie Diethylether, Diisopropylether, 1,2-Dimethoxy-ethan, Tetrahydrofuran und Dioxan, Ester, wie Essigsäuremethylester und -ethylester, Nitrile, wie Acetonitril und Propionitril, Amide, wie Dimethylformamid und Dimethylacetamid, sowie Dimethylsulfoxid und Sulfolan.

Die Umsetzung nach dem erfindungsgemäßen Verfahren (a) kann sowohl in Anwesenheit als auch in Abwesenheit von Säurebindemitteln vorgenommen werden. Arbeitet man in Gegenwart von Säurebindemitteln, so kommen als derartige Säureakzeptoren praktisch alle üblichen Säurebindemittel in Betracht.

Besonders bevorzugt verwendbar sind Alkalimetall- und Erdalkalimetallhydroxide, wie z.B. Natrium-, Kalium- und Calcium-hydroxid, Alkalimetall- und Erdalkalimetallcarbonate bzw. -hydrogen-carbonate, wie z.B. Natriumcarbonat und -hydrogencarbonat, Kaliumcarbonat und Calciumcarbonat, sowie aliphatische, aromatische und heterocyclische Amine, wie z.B. Trimethyl-, Triethyl-, Tripropyl- und Tributyl-amin, N,N-Dimethyl-benzylamin, Pyridin, 2-Methylpyridin, 2,4,6-Trimethyl-pyridin, 2-Methyl-5-ethyl-pyridin, Diazabicyclooctan (DABCO), Diazabicyclononen (DBN) und Diazabicycloundecen (DBU).

Die Reaktionstemperaturen können bei dem Verfahren (a) innerhalb eines größeren Bereiches variiert werden. Im allgemeinen arbeitet man bei Temperaturen zwischen -50°C und +200°C, vorzugsweise zwischen -20°C und +150°C.

Das Verfahren (a) wird im allgemeinen bei Normaldruck durchgeführt.

Zur Durchführung des erfindungsgemäßen Verfahrens (a) setzt man pro Mol an Ausgangsverbindung der Formel (II) im allgemeinen zwischen 0,8 und 4,0 Mol, vorzugsweise zwischen 0,9 und 3,0 Mol Ausgangsverbindung der Formel (III) ein. Arbeitet man in Gegenwart von Säurebindemitteln, so werden diese in äquivalenter Menge oder auch in einem Überschuß, bezogen auf die N-Chlorcarbonylverbindungen der Formel (II), eingesetzt. Die Durchführung der Umsetzung erfolgt beim Arbeiten ohne Säurebindemittel im allgemeinen in der Weise, daß man die Komponenten zusammengibt und dann bei erhöhter Temperatur reagieren läßt. Arbeitet man in Gegenwart eines Säurebindemittels, so werden die Komponenten gegebenenfalls unter Kühlung zusammengegeben, und das Reaktionsgemisch wird danach gegebenenfalls bei erhöhter Temperatur gerührt. Die anschließende Aufarbeitung erfolgt nach üblichen Methoden. Im allgemeinen geht man so vor, daß man den festen Rückstand abfiltriert, mit einem organischen Lösungsmittel extrahiert und die vereinigten organischen Phasen wäscht und einengt. Es ist jedoch auch möglich, das Reaktionsgemisch nach beendeter Umsetzung mit Wasser zu verdünnen, das entstehende Gemisch mit einem mit Wasser wenig mischbaren organischen Lösungsmittel zu extrahieren, die vereinigten organischen Phasen zu waschen und dann einzuengen. Die dabei anfallenden Produkte können durch übliche Maßnahmen, wie Umkristallisation oder auf chromatographischem Wege von eventuell noch enthaltenen Verunreinigungen befreit werden.

Die bei dem erfindungsgemäßen Verfahren (b) und (c) als Ausgangsstoffe benötigten Bis-chlorcarbonylaniline der Formel (IV) sind ebenfalls vorzugsweise durch diejenigen Reste definiert, die bereits im Zusammenhang mit den erfindungsgemäßen Stoffen der Formel (I) vorzugsweise für diese Reste genannt wurden.

Die Bis-chlorcarbonylaniline der Formel (IV) sind bekannt oder lassen sich nach bekannten Verfahren herstellen (vgl. z.B. G. Zumach, E. Kühle, Synthesis 1970, 542).

So erhält man die Bis-chlorcarbonylaniline der Formel (IV) beispielsweise dadurch, daß man nach DE-AS 1 018 054 zugängliche Thiokohlensäurealkylesterchloride erst mit entsprechenden Anilinen und dann mit Chlorcarbonylsulfenchlorid zu Dithiazolidindionen (vgl. G. Zumach, E. Kühle, Angew. Chemie 82 (1970) 66.) der Formel

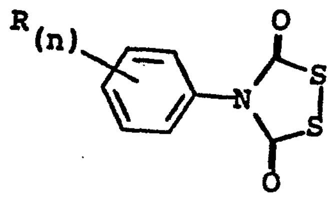

(VII)

umsetzt, in welchen

R und n die oben angegebene Bedeutung haben,

und dann die Dithiazolidindione der Formel (VII) einer chlorierenden Spaltung unterzieht, vorzugsweise mit Chlorierungsmitteln wie Sulfurylchlorid oder elementarem Chlor und gegebenenfalls in Lösungsmitteln wie Dichlormethan, Chloroform, Tetrachlorkohlenstoff, Chlorbenzol, Dichlorbenzol oder ähnlich inerten Solventien bei Temperaturen zwischen -20°C und +120°C.

Die gleichfalls als Ausgangsstoffe für das erfindungsgemäße Verfahren (b) und (c) geeigneten Bis-phenoxycarbonylaniline der Formel (IV) sind vorzugsweise durch diejenigen Substituenten festgelegt, deren Bedeutung bereits im Zusammenhang mit den erfindungsgemäßen Verbindungen der Formel (I) vorzugsweise für diese Reste genannt werden.

Die Bis-phenoxycarbonylaniline der Formel (IV) sind bekannt (vgl. z.B. EP-OS 48 376, 48 377 oder DE-OS 30 06 226) oder lassen sich mit den genannten Methoden auf einfache Art herstellen.

Beispielsweise erhält man die Verbindungen der Formel (IV), indem man Aniline der Formel (VIII)

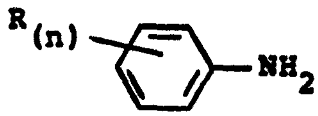

(VIII)

in welcher

R und n    die obengenannte Bedeutung haben,

oder deren Hydrohalogenide mit Chlorameisensäurephenylestern gegebenenfalls in einem Verdünnungsmittel wie vorzugsweise Toluol, Chlorbenzol oder Dichlorbenzol bei Temperaturen zwischen 0 und 200°C umsetzt. Die Verbindungen der Formel (VIII) sind bekannt.

Die ebenfalls als Ausgangsstoffe für das erfindungsgemäße Verfahren (b) erforderlichen Verbindungen der Formel (V) sind vorzugsweise durch diejenigen Reste gekennzeichnet, welche vorzugsweise im Zusammenhang mit den erfindungsgemäßen Stoffen der Formel (I) genannt wurden. Als Metallkation kommt vorzugsweise Lithium, Natrium, Kalium, Magnesium oder Calcium in Betracht. Es handelt sich um allgemein bekannte Verbindungen, ebenso wie bei den für die Durchführung des erfindungsgemäßen Verfahrens (b) ebenfalls erforderlichen Verbindungen der Formel (III), die bereits vorstehend vorzugsweise definiert sind.

Als Verdünnungsmittel können bei der Durchführung der erfindungsgemäßen Verfahren (b) und (c) alle inerten, aprotischen organischen Lösungsmittel verwendet werden. Vorzugsweise in Betracht kommen alle diejenigen Solventien, die bereits im Zusammenhang mit der Beschreibung des erfindungsgemäßen Verfahrens (a) vorzugsweise genannt wurden.

Falls das erfindungsgemäße Verfahren (b) bzw. (c) in Gegenwart von Hilfsbasen durchgeführt werden soll, so kommen vorzugsweise alle bei der Durchführung des erfindungsgemäßen Verfahrens (a) als Säureakzeptoren genannten Mittel in Betracht.

Die Reaktionstemperaturen können auch bei der Durchführung des erfindungsgemäßen Verfahrens (b) bzw. (c) innerhalb eines größeren Bereiches variiert werden. Im allgemeinen arbeitet man bei Temperaturen zwischen -20°C und 200°C, vorzugsweise zwischen 0°C und 150°C.

Das erfindungsgemäße Verfahren (b) bzw. (c) wird im allgemeinen unter Atmosphärendruck durchgeführt, kann aber auch vorteilhaft bei erhöhtem Druck bis zu 20 bar durchgeführt werden.

Bei der Durchführung der erfindungsgemäßen Verfahren (b) bzw. (c) setzt man die Komponente der Formel (IV) sowie die Reaktionspartner der Formel (V) und der Formel (III) im allgemeinen in äquivalenten Mengen ein. Es ist jedoch auch möglich, eine der jeweiligen Komponenten in einem Überschuß einzusetzen. Die Aufarbeitung erfolgt nach den üblichen Methoden, die bereits bei der Beschreibung der Durchführung des erfindungsgemäßen Verfahrens (a) genannt wurden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe können zur Bekämpfung von dikotylen und monokotylen Unkräutern in wichtigen Kulturen wie Zuckerrüben, Baumwolle und Sojabohnen eingesetzt werden.

Darüber hinaus weisen die erfindungsgemäßen Wirkstoffe

in entsprechenden Aufwandmengen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:

Botrytis-Arten, wie beispielsweise Botrytis cinerea;
Plasmopara-Arten, wie beispielsweise Plasmopara viticola;
Uromyces-Arten, wie beispielsweise Uromyces appendiculatus;
Sphaerotheca-Arten, wie beispielsweise Sphaerotheca fuliginea;
Venturia-Arten, wie beispielsweise Venturia inaequalis;
Podosphaera-Arten, wie beispielsweise Podosphaera leucotricha;
Phytophthora-Arten, wie beispielsweise Phytophthora infestans;

Erysiphe-Arten, wie beispielsweise Erysiphe graminis;
Puccinia-Arten, wie beispielsweise Puccinia recondita;
Fusarium-Arten, wie beispielsweise Fusarium culmorum;
Ustilago-Arten, wie beispielsweise Ustilago nuda oder avenae;
Septoria-Arten, wie beispielsweise Septoria nodorum;
Tilletia-Arten, wie beispielsweise Tilletia caries;
Xanthomonas-Arten, wie beispielsweise Xanthomonas oryzae;
Pseudomonas-Arten, wie beispielsweise Pseudomonas lachrymans;
Pyricularia-Arten, wie beispielsweise Pyricularia oryzae;
Pellicularia-Arten, wie beispielsweise Pellicularia sasakii;
Pyrenophora-Arten, wie beispielsweise Pyrenophora teres
(Konidienform: Drechslera, Syn: Helminthosporium);
Leptosphaeria-Arten, wie beispielsweise Leptosphaeria nodurum; Cochliobolus-Arten, wie beispielsweise Cochliobolus sativus
(Konidienform: Drechslera, Syn: Helminthosporium)
und
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Besonders hervorzuheben ist eine gute Wirkung gegen Pyricularia oryzae (protektiv und systemisch) am Reis, eine gute Wirkung gegen Getreiderost sowie eine gute Wirkung gegen Oomyceten und echte Mehltaupilze.

Die neuen Wirkstoffe eignen sich bei guter Pflanzenverträglichkeit und günstiger Warmblütertoxizität ferner auch zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blatella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malocosoma neustria, Euproctis chrysorhoea, Lymantria spp. Bucculatrix thurberiella, Phyllocnistis citrella, Agrotis spp., Euxoa spp., Feltia spp. Earias insulana, Heliothis spp., Laphygma exiqua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp.,

Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Neptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Besonders hervorzuheben ist eine gute Wirkung gegen Sproßinsekten.

Die neuen Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage:
Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glycol sowie deren Ether und Ester, Ketone wie Aceton,

Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und

Granulate angewandt werden. Die Anwendung geschieht in üblicher Weise, z.B. durch Gießen, Spritzen, Sprühen, Streuen. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die erfindungsgemäßen Wirkstoffe können bei der Herbizid-Anwendung sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen bei Herbizidanwendung zwischen 0,01 und 10 kg Wirkstoff pro Hektar Bodenfläche, vorzugsweise zwischen 0,05 und 5 kg pro ha.

Bei der Fungizid-Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Fungizid-Behandlung des Bodens sind Wirkstoffkonzentrationen von 0,00001 bis 0,1 Gew.-%, vorzugsweise von 0,0001 bis 0,02 %, am Wirkungsort erforderlich.

Der Wirkstoffgehalt der aus den handelsüblichen Insektizid-Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Insektizid-Anwendung geschieht in einer den Anwendungsformen angepaßten üblichen Weise.

Die nachfolgenden Beispiele dienen zur weiteren Erläuterung der Erfindung.

## Herstellungsbeispiele

### Beispiel 1

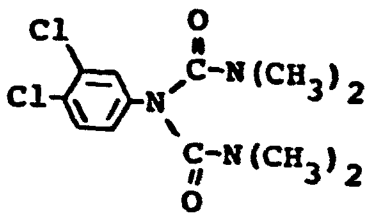

740 g (2,5 Mol) N-(3,4-Dichlorphenyl)-N-chlorcarbonyl-N',-N'-dimethylharnstoff werden in 2 l Toluol suspendiert. Dann leitet man bis zur Sättigung über Kaliumhydroxid getrocknetes gasförmiges Dimethylamin ein, wobei die Temperatur bei 20 bis 30°C gehalten wird. Anschließend wird mehrere Stunden nachgerührt, über Nacht stehengelassen, dann mit 2 l Wasser verrührt und das zweiphasige Gemisch abgesaugt und mit Wasser ausgewaschen. Zusammen mit dem nach Abtrennen des organischen Anteils des Filtrats und Einengen erhaltenen nachgefallenem Produktanteil ergeben sich 632 g farblose Kristalle vom Fp. 138-140°C (83 % der Theorie) an $N^1$-Dimethyl-$N^2$-(3,4-dichlorphenyl)-$N^3$-dimethylbiuret.

### Beispiel 2

8 g (0,08 Mol) 45 %ige wäßrige Dimethylaminlösung werden bei 0°C tropfenweise unter starkem Rühren mit einer Lösung von 8 g (0,027 Mol) N-(3,4-Dichlorphenyl)-N-chlorcarbonyl-N',N'-dimethylharnstoff in 30 ml Chloroform

versetzt. Man rührt bei Raumtemperatur einige Stunden nach, läßt über Nacht stehen, wäscht dann zweimal mit Wasser und engt nach Trocknen mit Natriumsulfat im Vakuum ein. Nach einigem Stehenlassen kristallisiert das anfangs ölige Produkt und man erhält 6,5 g (79 % der Theorie) der gleichen Verbindung wie in Beispiel 1, Fp.: 138-140°C.

Herstellung der Vorstufen:

a) 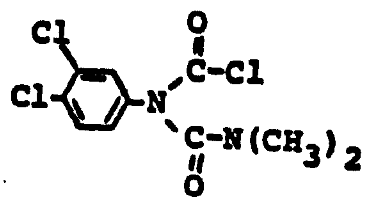

In eine Lösung von 720 g (2,91 Mol) O-Methyl-N-(3,4-dichlorphenyl)-N,N'-dimethyl-isoharnstoff in 2,2 l Chlorbenzol werden bei 0°C innerhalb 4 Stunden 420 g (Überschuß) Phosgen eingeleitet, wobei man später die Temperatur bis auf 80°C steigert. Anschließend wird für 3 Stunden (bis zum Ende der Gasentwicklung) auf 100°C aufgeheizt und danach im Vakuum das Lösungsmittel abdestilliert. Der ölige Rückstand wird mit Diisopropylether und Cyclohexan verrieben und nach Kristallisation abgesaugt. Man erhält 762 g farblose Kristalle (89 % der Theorie) an N-(3,4-Dichlorphenyl)-N-chlorcarbonyl-N',N'-dimethylharnstoff vom Fp. 79°C.

0192150

b)

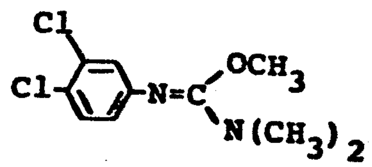

Zu einer Lösung von 1338 g (5,5 Mol) 3,4-Dichlorphenylisocyaniddichlorid (vgl. E. Kühle et al., Angew. Chem. 79 (1967) 678) in 4 l Toluol werden bei 28°C 220 g (5,5 Mol) Natriumhydroxid, gelöst in 220 ml Wasser und 700 ml Methanol (Überschuß), langsam zudosiert und über Nacht bei Raumtemperatur nachgerührt. Nach viermaligem Auswaschen der Toluolphase mit Wasser wird diese mit 1,2 l 40 %iger wäßriger Dimethylaminlösung (Überschuß) verrührt und am folgenden Tag wiederum mit Wasser gewaschen und nach Trocknen über Natriumsulfat und Eindampfen im Vakuum mit einem Gemisch aus Diisopropylether und Cyclohexan verrieben. Dabei kristallisieren 749 g (55 % der Theorie) des farblosen O-Methyl-N-(3,4-dichlorphenyl)-N'-dimethylisoharnstoff aus vom Fp. 52-53°C (Verfahren nach DE-Patent 1126380 bzw. Angew. Chem. 81 (1969), 22).

Beispiel 3

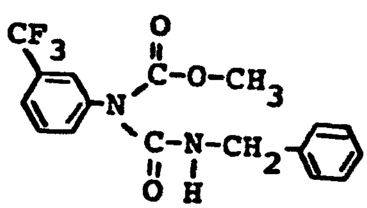

Zu einer Lösung von 3,4 g (0,032 Mol) Benzylamin in 100 ml Dichlormethan und 2,5 g (0,032 Mol) Pyridin

tropft man bei 0°C 9 g (0,032 Mol) N-(3-Trifluormethylphenyl)-N-chlorcarbonyl-methylurethan, gelöst in 50 ml Dichlormethan, läßt über Nacht stehen und erhitzt dann 10 Stunden unter Rückfluß zum Sieden. Nach Aufarbeitung wie in Beispiel 2 erhält man 9,9 g (88 % der Theorie) an N-(3-Trifluormethylphenyl)-N-methoxycarbonyl-N'-benzylharnstoff in Form eines hochviskosen Öls (IR-Banden: NH 3300 $cm^{-1}$, C=O 1680, 1720 $cm^{-1}$).

Herstellung der Vorstufen

a) 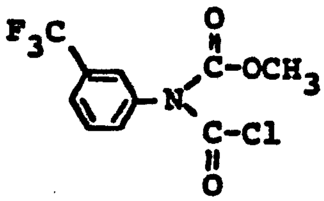

In eine Lösung von 197 g (0,88 Mol) N-3-Trifluormethylphenyliminokohlensäuredimethylester in 500 ml Chlorbenzol wird bei 0 bis 10°C im Überschuß Phosgen eingeleitet, danach bis auf Siedetemperatur erhitzt und dann das verbliebene Phosgen mit Stickstoff ausgeblasen. Nach Abziehen des Lösungsmittels im Vakuum und Abkühlen des verbliebenen viskosen Öls kristallisiert dieses und nach Verreiben mit Petrolether können 218 g (88 % der Theorie) an N-(3-Trifluormethylphenyl)-N-chlorcarbonyl-urethan vom Fp. 102°C abgesaugt werden.

b) 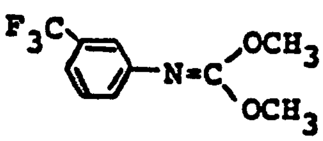

In eine Natriummethylatlösung, frisch hergestellt aus 43 g Natrium durch Auflösung in 800 ml Methanol (entsprechend 1,86 Mol) werden 225 g (0,93 Mol) 3-Trifluormethyl-phenylisocyanid-dichlorid (Herstellung analog E. Kühle, Angew. Chem. 79 (1967) 678, Sdp. 64°C/0,35 mbar) getropft, wobei man die Temperatur bis 55°C steigen läßt. Nach einer Stunde wird filtriert und das Filtrat destilliert, wobei bei 110°C/13 mbar 210 g (97 % der Theorie) an N-(3-Trifluormethylphenyl)-iminokohlensäuredimethylester in Form eines farblosen Öls übergehen.

Die im folgenden in der Tabelle 1 aufgeführten Beispiele lassen sich in entsprechender Weise synthetisieren.

Tabelle 1 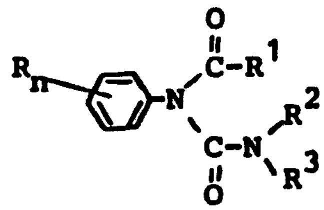 (I)

| Beispiel | $R_n$ | $R^1$ | 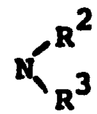 | Fp. (°C) bzw. Brechungsindex |
|---|---|---|---|---|
| 4 | H | $OCH_3$ | $NHCH_3$ | 116-117 |
| 5 | H | " | $N(CH_3)_2$ | 84-85 |
| 6 | H | " | $NHC_2H_5$ | 97-98 |
| 7 | 4-$OCF_3$ | " | $N(CH_3)_2$ | $n_D^{20}$ 1,478 |
| 8 | 2-$OCF_3$ | " | " | 1,479 |
| 9 | 2-$CH_3$, 4-Cl | " | " | visk. Öl |
| 10 | " | " | $N(C_2H_5)_2$ | 84-88 |
| 11 | " | " | $N(C_3H_7)_2$ | visk. Öl |
| 12 | 3-Cl, 4-$CH_3$ | " | $N(CH_3)_2$ | " |
| 13 | " | $OC_2H_5$ | " | " |
| 14 | " | " | $N(C_2H_5)_2$ | " |
| 15 | " | " | $N(C_3H_7)_2$ | " |
| 16 | 2-Cl, 5-$CF_3$ | $N(CH_3)_2$ | $N(CH_3)_2$ | 159-161 |
| 17 | 2-$CF_3$, 4-Cl | " | " | $n_D^{20}$ 1,529 |
| 18 | " | " | 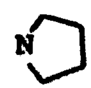 | 1,538 |

| Beispiel | $R_n$ | $R^1$ | $N{<}^{R^2}_{R^3}$ | Fp. (°C) bzw. Brechungsindex |
|---|---|---|---|---|
| 19 | 3-$CF_3$ | $OC_2H_5$ | $N(CH_3)_2$ | $n_D^{20}$ 1,475 |
| 20 | " | " | $N(C_2H_5)_2$ | visk. Öl |
| 21 | " | " | $N(CH_2CH{=}CH_2)_2$ | " |
| 22 | " | " | $N(C_3H_7)_2$ | " |
| 23 | " | $OCH_2C{\equiv}CH$ | $N(CH_3)_2$ | 56-58 |
| 24 | " | " | $N(CH_2CH{=}CH_2)_2$ | visk. Öl |
| 25 | " | $OC_{10}H_{21}(n)$ | $N(CH_3)_2$ | $n_D^{20}$ 1,471 |
| 26 | " | " | 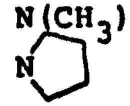 | 1,484 |
| 27 | " | $OCH_2CH_2OCH_3$ | " | 54-56 |
| 28 | " | $SCH_3$ | $N(CH_2CH{=}CH_2)_2$ | visk. Öl |
| 29 | " | $SC_4H_9$ | $N(CH_3)_2$ | " |
| 30 | " | " | $N(C_2H_5)_2$ | " |
| 31 | " | " | $N(C_3H_7)_2$ | " |
| 32 | " | " | $N(CH_2CH{=}CH_2)_2$ | " |
| 33 | " | $OCH_3$ | $NHCH_3$ | 110 |
| 34 | " | " | $N(CH_3)_2$ | $n_D^{20}$ 1,485 |
| 35 | " | " | $N(C_2H_5)_2$ | visk. Öl |
| 36 | " | " | $NHCH(CH_3)_2$ | 104-105 |
| 37 | " | " | $N(CH(CH_3)_2)_2$ | visk. Öl |
| 38 | " | " | $N(CH_2CH{=}CH_2)_2$ | $n_D^{20}$ 1,492 |
| 39 | " | " | 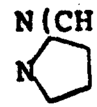 | 1,496 |
| 40 | " | " | 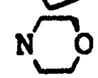 | 1,492 |

| Beispiel | $R_n$ | $R^1$ | 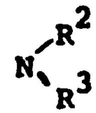 | Fp. (°C) bzw. Brechungsindex |
|---|---|---|---|---|
| 41 | 3-$CF_3$ | $NHCH_3$ | $N(CH_3)_2$ | visk. Öl |
| 42 | " | $N(CH_3)_2$ | " | 140 |
| 43 | " | $NHC(CH_3)_3$ | " | $n_D^{20}$ 1,479 |
| 44 | " | $NHCH_2C(CH_3)_3$ | " | 1,482 |
| 45 | " | 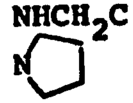 | " | 145-147 |
| 46 | " | 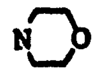 | " | 100 |
| 47 | " | $NHCH_3$ | 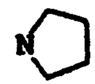 | 100-106 |
| 48 | " | $NHCH(CH_3)_2$ | " | 95-97 |
| 49 | " | 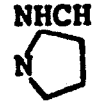 | $N(CH_2CH{=}CH_2)_2$ | $n_D^{20}$ 1,513 |
| 50 | " | " | 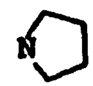 | 183-185 |
| 51 | " | " | 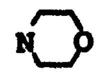 | 155 |
| 52 | 2,5-Di-Cl | $OCH_3$ | $N(C_2H_5)_2$ | 82-85 |
| 53 | 4-Cl | " | $N(CH_3)_2$ | visk. Öl |
| 54 | " | $OC_2H_5$ | " | 64-65 |
| 55 | " | $OCH(CH_3)_2$ | " | 76-78 |
| 56 | " | $N(CH_3)_2$ | " | 145 |
| 57 | " | 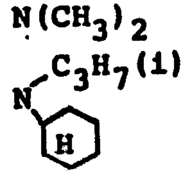 | 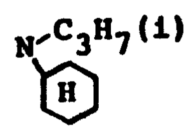 | 162-163 |

| Beispiel | $R_n$ | $R^1$ | 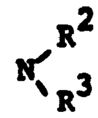 | Fp. (°C) bzw. Brechungsindex |
|---|---|---|---|---|
| 58 | 3,4-Di-Cl | $OCH_3$ | $NHCH_3$ | 145-147 |
| 59 | " | " | $N(CH_3)_2$ | 86-88 |
| 60 | " | " | $NHC_2H_5$ | 157-158 |
| 61 | " | " | $N(C_2H_5)_2$ | 44-46 |
| 62 | " | " | $N(C_3H_7)_2$ | visk. Öl |
| 63 | " | " | $N(CH_2CH{=}CH_2)_2$ | " |
| 64 | " | " | 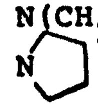 | 128 |
| 65 | " | $OC_2H_5$ | $N(CH_3)_2$ | $n_D^{20}$ 1,530 |
| 66 | " | " | $N(C_2H_5)_2$ | visk. Öl |
| 67 | " | " | $N(C_3H_7)_2$ | " |
| 68 | " | " | $N(CH_2CH{=}CH_2)_2$ | " |
| 69 | " | $OCH(CH_3)_2$ | $N(CH_3)_2$ | " |
| 70 | " | " | $N(C_2H_5)_2$ | 92-94 |
| 71 | " | " | $N(C_3H_7)_2$ | visk. Öl |
| 72 | " | " | $N(CH_2CH{=}CH_2)_2$ | " |
| 73 | " | $OCH_2C{\equiv}CH$ | $N(CH_3)_2$ | " |
| 74 | " | " | $N(C_2H_5)_2$ | " |
| 75 | " | " | $N(CH_2CH{=}CH_2)_2$ | " |
| 76 | " | $OC_4H_9$ | $N(CH_3)_2$ | $n_D^{20}$ 1,510 |
| 77 | " | $OCH_2CH_2OCH_3$ | " | 1,523 |
| 78 | " | $SCH_3$ | " | 60-62 |
| 79 | " | " | $N(C_2H_5)_2$ | visk. Öl |

| Beispiel | $R_n$ | $R^1$ | 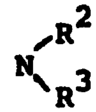 | Fp. (°C) bzw. Brechungsindex |
|---|---|---|---|---|
| 80 | 3,4-Di-Cl | $SC_3H_7$ | $N(CH_3)_2$ | visk. Öl |
| 81 | " | " | $N(C_2H_5)_2$ | " |
| 82 | " | 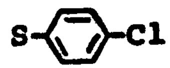 | $N(CH_3)_2$ | 122-125 |
| 83 | " | $NHCH_3$ | $NHCH_3$ | 143-144 |
| 84 | " | $NHCH_2C(CH_3)_3$ | $N(CH_3)_2$ | 92 |
| 85 | " | $NHC_{12}H_{25}(n)$ | " | $n_D^{20}$ 1,510 |
| 86 | " | 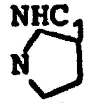 | " | 139-140 |
| 87 | " | 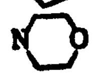 | " | 108 |
| 88 | " | 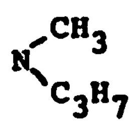 | 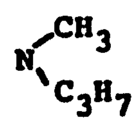 | visk. Öl |
| 89 | " | $N(C_3H_7)_2$ | $N(C_3H_7)_2$ | " |
| 90 | " | 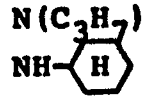 | 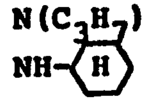 | 134-135 |
| 91 | 2,4,5-Tri-Cl | $OCH_3$ | $N(CH_3)_2$ | visk. Öl |
| 92 | " | " | $N(C_2H_5)_2$ | " |
| 93 | " | $OC_2H_5$ | $N(CH_3)_2$ | " |
| 94 | " | " | $N(C_2H_5)_2$ | " |
| 95 | " | " | $N(C_3H_7)_2$ | " |

| Beispiel | $R_n$ | $R^1$ | 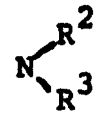 | Fp. (°C) bzw. Brechungsindex |
|---|---|---|---|---|
| 96 | 2,4,5-Tri-Cl | $OCH_2C{\equiv}CH$ | $N(CH_3)_2$ | 105-108 |
| 97 | " | " | $N(C_2H_5)_2$ | 64-65 |
| 98 | " | $SCH_3$ | $N(CH_3)_2$ | visk. Öl |
| 99 | " | " | $N(C_2H_5)_2$ | " |
| 100 | $3-CF_3$ | $OCH_3$ | 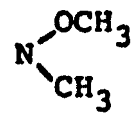 | " |
| 101 | " | 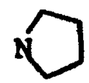 | " | " |
| 102 | 3,4-Di-Cl | $N(CH_3)_2$ | " | " |

0192150

## Anwendungsbeispiele

### Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit gegenüber dem Stand der Technik zeigt in diesem Test z.B. die Verbindung gemäß dem Herstellungsbeispiel: 1.

Als Vergleichsmittel diente DIURON /N-(3,4-Dichlorphenyl)-N',N'-dimethylharnstoff/.

**Beispiel B**

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit gegenüber dem Stand der Technik zeigen in diesem Test z.B. die Verbindungen gemäß den Herstellungsbeispielen:76, 77.

Als Vergleichsmittel diente ebenfalls DIURON.

**Beispiel C**

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen: 17, 19, 39, 87.

Beispiel D

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeispielen:
14, 37, 69, 85.

## Beispiel E

Puccinia-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z.B. die Verbindungen gemäß den Herstellungsbeispielen:
1, 65, 76, 77.

**Beispiel F**

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle

Testtiere abgetötet sind und 0 %, wenn noch genau so viele Testinsekten leben wie bei der unbehandelten Kontrolle.

Bei diesem Test zeigen z.B. die Verbindungen der Herstellungsbeispiele überlegene Wirkung gegenüber dem Stand der Technik: 33, 41, 47, 76, 84.

**Beispiel G**

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle

Testtiere abgetötet sind und 0 %, wenn noch genau so viele Testinsekten leben wie bei der unbehandelten Kontrolle.

Bei diesem Test zeigen z.B. die Verbindungen der Herstellungsbeispiele überlegene Wirkung gegenüber dem Stand der Technik: 41, 84.

Patentansprüche

1. 1-Acyl-1-phenyl-harnstoffe der allgemeinen Formel

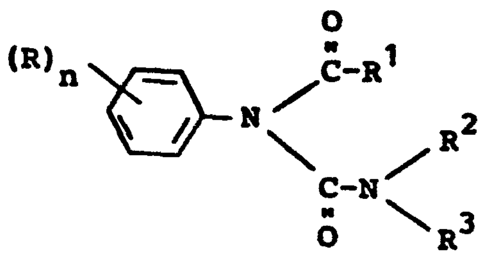

(I)

in welcher die Reste

R gleich oder verschieden sind und für Halogen, Alkyl, Alkoxy, Halogenalkyl, Halogenalkoxy, Alkylthio, Halogenalkylthio oder Nitro stehen,

n für 0, 1, 2, 3, 4 oder 5 steht,

$R^1$ für Alkoxy, Alkenoxy, Alkinoxy, Alkoxyalkoxy, Alkylthio, Arylthio, Alkylamino, Dialkylamino, Cycloalkylamino oder für einen über Stickstoff gebundenen N-Heterocyclus steht,

$R^2$ für Wasserstoff, Alkyl, Alkenyl oder Alkoxy steht und

$R^3$ für Alkyl, Alkenyl, Cycloalkyl oder Aralkyl steht oder

$R^2$ und $R^3$ gemeinsam mit dem Stickstoffatom, an das sie gebunden sind, für einen heterocyclischen

Rest stehen, und in welcher der Rest $R^1$ mit dem Rest

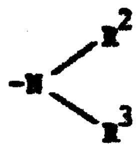 identisch sein kann.

2. 1-Acyl-1-phenyl-harnstoffe der allgemeinen Formel (I) gemäß Anspruch 1, in welcher

R für Fluor, Chlor, Brom, geradkettiges oder verzweigtes Alkyl mit bis zu 12 C-Atomen, geradkettiges oder verzweigtes Alkoxy mit 1 bis 4 Kohlenstoffatomen, Halogenalkyl mit 1 bis 4 C-Atomen und einem oder mehreren F-, Cl- oder Br-Atomen, Halogenalkoxy mit 1 bis 4 C-Atomen und bis zu 9 gleichen oder verschiedenen Halogenatomen F, Cl, Br; Halogenalkylthio mit 1 bis 4 C-Atomen und gleichen oder verschiedenen Halogensubstituenten F, Cl, Br; oder für Nitro steht,

n für 0 bis 5 steht,

$R^1$ für Alkoxy ($C_1$-$C_{12}$), Alkenoxy ($C_2$-$C_4$), Alkinoxy ($C_2$-$C_4$), Alkoxyalkoxy mit jeweils 1 bis 4 C-Atomen im Alkoxyteil, Alkylthio ($C_1$-$C_6$), gegebenenfalls durch Chlor oder Methyl substituiertes Phenylthio, geradkettiges oder verzweigtes Alkylamino mit 1 bis 12 C-Atomen,

geradkettiges oder verzweigtes Dialkylamino mit 1 bis 12 C-Atomen, wobei bei den Alkylamino- und Dialkylaminoderivaten auch ein oder beide Alkylreste einen carbocyclischen Ring mit 5 bis 7 C-Atomen bilden können, für einen über Stickstoff gebundenen N-Heterocyclus mit 4 bis 8 Ringgliedern, die gegebenenfalls durch ein Sauerstoffatom unterbrochen sind, steht,

$R^2$ für Wasserstoff, Alkyl ($C_1$-$C_{12}$), Alkenyl ($C_1$-$C_4$) oder Alkoxy ($C_1$-$C_4$) steht,

$R^3$ für Alkyl ($C_1$-$C_{12}$), Alkenyl ($C_1$-$C_4$), Cycloalkyl mit 4 bis 8 Ring-C-Atomen, Arylalkyl mit 1 bis 5 C-Atomen im Alkylteil und 6 C-Atomen im Arylteil steht,

oder wobei $R^2$ und $R^3$ gemeinsam mit dem Stickstoffatom, an das sie gebunden sind, für einen N-Heterocyclus mit 4 bis 8 Ringgliedern, die gegebenenfalls auch Sauerstoff enthalten können, stehen.

3. 1-Acyl-1-phenyl-harnstoffe der allgemeinen Formel (I) gemäß Anspruch 1, in welcher

R Wasserstoff, Fluor, Chlor, Brom, Methyl, Ethyl, Propyl, Isopropyl, Methoxy, Ethoxy, Propoxy, Isopropoxy, $CH_2Cl$, $CCl_3$, $CFCl_2$, $CF_2Cl$, $CF_3$, $OCF_3$, $SCF_3$ oder $NO_2$ bedeutet,

$R^1$ geradkettiges oder verzweigtes Alkoxy ($C_1$-$C_{10}$), Allyloxy, Propargyloxy, Alkoxy($C_1$-$C_3$)alkoxy ($C_1$-$C_2$), Alkylthio ($C_1$-$C_4$), Phenylthio, 4-Methylphenylthio, 4-Chlorphenylthio, Alkylamino ($C_1$-$C_{12}$), Cyclopentyl, Cyclohexyl, Pyrrolidino, Piperidino, Hexamethylenimino oder Morpholino bedeutet,

$R^2$ Wasserstoff, Methyl, Ethyl, Propyl, Isopropyl, Butyl, Isobutyl, tert.-Butyl, Pentyl, Isopentyl, Neopentyl, Allyl, Methoxy oder Ethoxy bedeutet,

$R^3$ Methyl, Ethyl, Propyl, Isopropyl, Butyl, Isobutyl, tert.-Butyl, Pentyl, Isopentyl, Neopentyl, Allyl, Methoxy, Ethoxy, Cyclopentyl oder Cyclohexyl bedeutet und

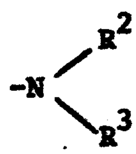 Pyrrolidino, Piperidino, Hexamethylenimino oder Morpholino bedeutet.

4. Verfahren zur Herstellung von 1-Acyl-1-phenylharnstoffen der allgemeinen Formel (I)

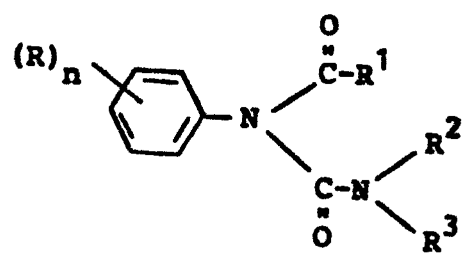

(I)

in welcher die Reste

R gleich oder verschieden sind und für Halogen, Alkyl, Alkoxy, Halogenalkyl, Halogenalkoxy, Alkylthio, Halogenalkylthio oder Nitro stehen,

n für 0, 1, 2, 3, 4 oder 5 steht,

$R^1$ für Alkoxy, Alkenoxy, Alkinoxy, Alkoxyalkoxy, Alkylthio, Arylthio, Alkylamino, Dialkylamino, Cycloalkylamino oder für einen über Stickstoff gebundenen N-Heterocyclus steht,

$R^2$ für Wasserstoff, Alkyl, Alkenyl oder Alkoxy steht und

$R^3$ für Alkyl, Alkenyl, Cycloalkyl oder Aralkyl steht oder

$R^2$ und $R^3$ gemeinsam mit dem Stickstoffatom, an das sie gebunden sind, für einen heterocyclischen Rest stehen, und in welcher der Rest $R^1$ mit dem Rest

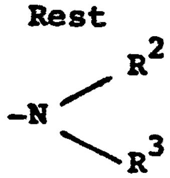 identisch sein kann,

dadurch gekennzeichnet, daß man

(a) N-Acyl-N-chlorcarbonyl-aniline der Formel (II)

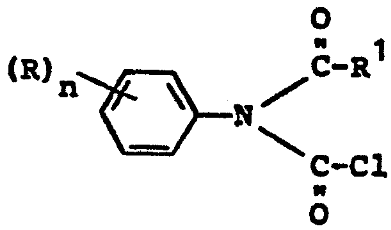

(II)

in welcher

R, n und $R^1$ die oben angegebene Bedeutung besitzen,

mit Aminen der Formel (III)

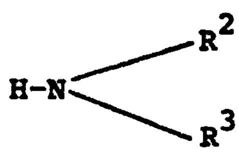

(III)

in welcher

$R^2$ und $R^3$ die oben angegebene Bedeutung haben,

gegebenenfalls in Gegenwart eines Verdünnungsmittels und gegebenenfalls in Gegenwart eines Säurebindemittels umsetzt,

oder

(b) zur Herstellung von Verbindungen der Formel (I), in welcher die Reste $R^1$ und

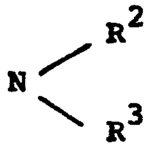 voneinander verschieden sind, ein Mol

einer N-Phenylimido-dicarbonylverbindung der Formel (IV)

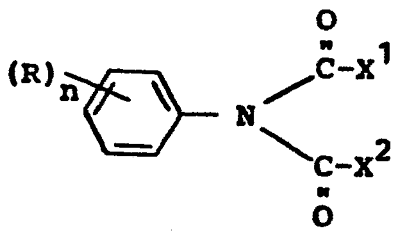

(IV)

in welcher

R und n die oben angegebene Bedeutung haben und

$X^1$ und $X^2$ gleich oder verschieden sein können und für Halogen oder Phenoxy stehen,

gegebenenfalls in Gegenwart eines Verdünnungsmittels und gegebenenfalls in Gegenwart einer Hilfsbase in beliebiger Reihenfolge mit etwa einem Mol einer Verbindung der Formel

$$M\text{-}R^1 \qquad (V)$$

in welcher

$R^1$ die oben angegebene Bedeutung hat und

M für Wasserstoff oder ein Äquivalent eines Metallkations steht,

und etwa einem Mol einer Verbindung der Formel (III)

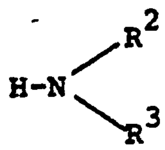

(III)

worin

$R^2$ und $R^3$ die oben angegebene Bedeutung haben,

umsetzt,

oder

(c) zum Erhalt von Verbindungen der Formel (I), in welcher der Rest $R^1$ mit dem Rest

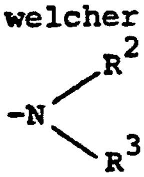

identisch ist,

ein Mol einer Verbindung der Formel (IV)

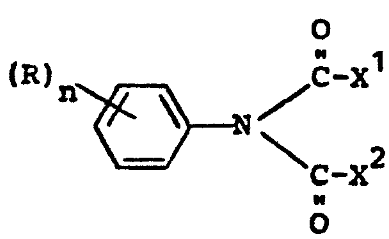

(IV)

in welcher

R, n, $X^1$ und $X^2$ die oben angegebene Bedeutung besitzen,

mit mindestens zwei Mol einer Verbindung der Formel (III)

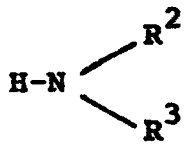

(III)

worin

$R^2$ und $R^3$ die oben angegebene Bedeutung haben,

gegebenenfalls in Gegenwart eines Verdünnungsmittels und gegebenenfalls in Gegenwart eines Säurebindemittels umsetzt.

5. Pflanzenschutz- bzw. Pflanzenbehandlungsmittel, gekennzeichnet durch einen Gehalt an mindestens einem 1-Acyl-1-phenyl-harnstoff der Formel (I) gemäß Anspruch 1.

6. Verwendung von 1-Acyl-1-phenyl-harnstoffen der Formel (I) gemäß Anspruch 1 als Pflanzenschutz- bzw. Pflanzenbehandlungsmittel, insbesondere zur Bekämpfung von unerwünschtem Pflanzenwachstum, zur Bekämpfung von unerwünschten Mikroorganismen und von schädlichen Insekten, jeweils in geeigneten Konzentrationen bzw. Aufwandmengen.

7. Verfahren zur Herstellung von Pflanzenschutz- bzw. Pflanzenbehandlungsmitteln, dadurch gekennzeichnet, daß man 1-Acyl-1-phenyl-harnstoffe der Formel (I) gemäß Anspruch 1 mit Streckmitteln und/oder oberflächenaktiven Mitteln vermischt.